# EUROPEAN PATENT SPECIFICATION

(11) **EP 0 891 783 B1**
(45) Date of publication and mention of the grant of the patent: **12.06.2002**
(21) Application number: 98202360.8
(22) Date of filing: 14.07.1998
(51) Int. Cl.: A61L 27/00

(54) **Device for tissue engineering bone comprising biodegradable thermoplastic copolyester and cultured cells**
Vorrichtung zur Knochenbehandlung bestehend aus abbaubarem thermoplastischen Copolyester und kultivierten Zellen
Dispositif pour la régénération osseuse consistant d'un copolyester thermoplastique biodégradable et de cellules cultivées

(30) Priority: 16.07.1997 EP 97202214
(43) Date of publication of application: 20.01.1999
(73) Proprietor: IsoTis N.V., 3723 MB Bilthoven (NL)
(72) Inventor: de Bruijn, Joost Dick, 2517 AG Den Haag (NL); Bovell, Yvonne Pearl, 2517 AG Den Haag (NL); van den Brink, Jennigje, 2341 SR Oegstgeest (NL); van Blitterswijk, Clemens Antoni, 3476 PD Hekendorp (NL)
(74) Representative: Renes, Johan

(56) References cited:
- EP-A- 0 357 155
- EP-A- 0 705 609
- WO-A-93/07916
- WO-A-95/03011
- WO-A-96/28539
- US-A- 5 489 306
- RADDER A.M. ET AL: 'Bone-bonding behaviour of poly(ethylene oxide)-polybutylene terephthalate copolymer coatings and bulk implants: a comparative study' BIOMATERIALS vol. 16, no. 7, 01 January 1995, pages 507 - 513

## Description

The invention relates to a device facilitating cell growth, differentiation and subsequent osseous tissue generation in vitro and later in vivo, which comprises a porous, bioactive, osteoconductive and bone-bonding, polymer.

### Background

US Patent 5,226,914 (AI Caplan) discloses a method for treating connective tissue disorders by isolating and culturally expanding marrow-derived mesenchymal stem cells, adhering the cells onto the surface of a prosthetic device and implanting the prosthetic device containing the culturally expanded cells into the type of skeletal or connective tissue needed for implantation.

US Patent 5,399,665 (D Barrera) discloses the synthesis and applications of a hydrolytically degradable polymer useful in biomedical applications involving the interaction of cells with the polymer structure, by coupling peptides to the free amino groups of the polymers.

US Patent 5,041,138 (JP Vacanti) discloses methods and artificial matrices for the growth and implantation of cartilaginous structures and surfaces and the production of bioactive molecules manufactured by chondrocytes. Chondrocytes are grown in culture on biodegradable, biocompatible fibrous matrices until an adequate cell volume and density has developed for the cells to survive and proliferate in vivo, and the matrices are designed to allow adequate nutrient and gas exchange to the cells until engraftment and vascularisation at the site of engraftment occurs.

WO 95/03011 discloses a biodegradable prosthetic template of a degradable polymer such as poly(lactic acid) or poly(lactic-co-glycolic acid) with a pore-former such as salt or gelatin, which template may be seeded with osteoblasts. The polymers used do not bind to bone and the osteoblasts are highly differentiated cells.

WO 96/28539 proposes a composition for growing cartilage or bone consisting of a biodegradable polymeric carrier such as polyglycolic acid or a polysaccharide containing mesenchymal stem cells. Mesenchymal stem cells are cells which are pluripotent, i.e. which can differentiate to various tissue types (muscle, cartilage, skin), while the polymers proposed do not bind to bone. EP 0 357 155 discloses films of segmented polyoxyethylene/polybutylene terephthalate copolyester-ethers with 35-70% by weight of long-chain ester units, on which middle ear epithelium cells are grown.

These prior art methods involve cells that are grown in the materials for the purpose of expansion or proliferation after which the materials containing the culturally expanded cells, are implanted at the site of engraftment. The prior art materials are degradable matrices, whether or not designed to couple peptides or biologically active moieties to serve to enhance binding of cells to the polymer, that mainly function as temporary devices for cell attachment. These prior methods therefore necessitate the production of connective tissues in vivo, while the prior materials function as a carrier for cell attachment and cell growth.

Surgical procedures related to bone tissue deficiencies vary from joint replacement, bone grafting and internal fixation, to maxillo-facial reconstructive surgery. From a biological perspective, the ideal material to reconstruct osseous tissues is autogenous bone, because of its compatibility, osteoinductivity, osteoconductivity, and lack of immunologic response. However, the limitations of harvesting an adequate amount of autogenous bone, and the disadvantages of a secondary operation to harvest the autologous bone, make this "ideal" material far from ideal for many surgical procedures.

Alternatives are other bone-derived materials and man-made biomaterials. The first group concerns allogeneic and xenogeneic bone grafts. A problem is, that they exhibit the possibility of disease transfer such as HIV or hepatitis B, a higher immunogenic response, less revascularisation of the graft and manifest unreliable degradation characteristics.

The second group concerns man-made, alloplastic implant materials, or biomaterials, which are readily available in large quantities. The wide variety of biomaterials that are used in clinical applications can be divided into four major categories: metals, ceramics, polymers and composites, which all have their own characteristics. The most interesting alloplastic biomaterials for bone replacement are bioactive or osteoconductive materials, which means that they can bind to bone tissue. Bioactive materials can be found in all four of the above mentioned biomaterials categories and include polymers such as PEO/PBT copolymers, calcium phosphate ceramics such as hydroxyapatite and bioglasses or glass-ceramics.

Compared to autogenous bone, the main disadvantage of biomaterials is that, without added osteoinductive agents such as bone morphogenetic proteins, they are not osteoinductive and therefore do not have the ability to actively induce bone formation. Although this can be overcome by adding osteoinductive growth factors to the materials, difficulties still exist to gradually release these factors from the biomaterial surface over a prolonged time period, which is needed to have a sufficient biological response.

This is why there is a need for another approach for the treatment of osseous defects, which combines cultured autogenous tissues with biomaterials, in so-called biomaterial-tissue hybrid structures. Although the combination of cultured cells with biomaterials to form biomaterial-cell composites may also be advantageous in that the cultured cells, after implantation, can give rise to the formation of a tissue, we describe herein an invention of a device in which cells are cultured to produce an extracellular matrix, after which this biomaterial-tissue hybrid is implanted at the site of engraftment.

### Description of the invention

The present invention concerns a device made up from a polymeric material that is biocompatible, osteoconductive and bone-bonding (bioactive), that can be used to culture undifferentiated, differentiated, osteogenic or (osteo)progenitor cells that form a bone-like extracellular matrix in vitro, after which the polymer containing the biological extracellular matrix is placed or implanted at the site of engraftment. The uniqueness about the present invention is two-fold. In a first aspect, in contrast to the prior art methods, the material can calcify by itself during immersion in cell culture medium or post-operatively, or can be pre-calcified, thereby exhibiting bioactive and osteoconductive or bone-bonding properties that will improve tissue-material interaction. In the second aspect, undifferentiated, differentiated, osteogenic or (osteo)progenitor cells are grown in the bioactive, biodegradable polymeric matrix not only to expand, but to actively produce an extracellular matrix in vitro. Consequently, a hybrid structure encompassing a bioactive, biodegradable polymeric matrix and an already in vitro formed biological extracellular matrix is produced that can be used for engraftment in osseous defects or at sites where bone is needed. This invented hybrid structure can be seen as a flexible autogenous cultured bone graft which is unique.

The polymeric matrix can be constituted of a segmented thermoplastic bioactive, preferably biodegradable polymer, such as described in EP-A-357155 or WO-93/21858. The molecules of such segmented thermoplastic copolyester (polyesterether) consist essentially of segments of recurring long-chain ester units and segments with recurring short-chain ester units. The long-chain ester units preferably comprise 35-80% by weight of the copolyester (polyether) being represented by the formula

-OLO-CO-R-CO-

and the short chain ester units being represented by the formula

-OEO-CO-R-CO-

wherein
- L: is a divalent group remaining after removal of terminal hydroxyl groups from a poly(oxyalkylene) glycol with an average molecular weight of between 300 and 500 or between 500 and 3000;
- R: is a divalent group remaining after removal of carboxyl groups from a dicarboxylic acid having a molecular weight of less than 300; and
- E: is an alkylene group having 2-6 carbon atoms.

Examples of the alkylene group in the poly(oxyalkylene) glycol of L include ethylene, 1,2-propylene, 2-hydroxy-1,3-propylene and butylene, poly(oxyethylene) glycol being preferred, especially polyoxyethylene on average consisting of 7 or 8 or more, up to about 70 oxyethylene units. The dicarboxylic acid of R may be aliphatic such as succinic, maleic, fumaric, glutaric, adipic, or preferably alicyclic such as cyclohexanedicarboxylic, but most preferably it is aromatic such as isophthalic and terephthalic acid, which may be ring-substituted by e.g. hydroxy, alkyl, alkoxy, halogen and acyloxy, as well as dicarboxylic acids of, optionally ring-substituted, naphthalene, tetraline, biphenyl, diphenylmethane, diphenyl ether or diphenyl sulphone; terephthalic acid is especially preferred. A preferred example of alkylene in E is 1,4-butylene.

Alternatively, the molecules of such segmented thermoplastic copolyester consist of two or three blocks, one of these blocks being represented by the formula -O-L-, -O-L-O-CO- or -O-L-O-CO-R-CO- and the other blocks containing recurring units represented by the formula -O-Q-CO- and optionally units represented by the formula -O-E-O-CO-R-CO-, wherein Q is an alkylene group having 1-6 carbon atoms optionally linked or a cyclohexylene or phenylene group or a comination thereof. Examples of the groups Q include methylene, ethylidene, ethylene, 1,3-propylene, 1,5-pentylene, 1,4-cyclohexylene, p-phenylenemethylene and p-ethylenephenylene.

According to one embodiment of the invention, the scaffold material is formed as a flexible sheet or structure. According to another embodiment, the material is formed as a rigid sheet or structure. The scaffold material may also adbvantageously be formed from weaved or intertwined fibres by a weaving, compressing or sintering process. The device may further suitably comprise filler materials such as calcium phosphates, bioactive glasses or glass ceramics, in needle-shaped, fibre or granular form. The device of the invention and the method of producing it are further defined in the appending claims.

An advantage of the present technique is not only that a more organised tissue is produced in vitro prior to its implantation in vivo, which may give rise to a more rapid healing, but also that the implanted biomaterial-tissue hybrid exists of a surface that is covered by an extracellular matrix produced by the cultured cells. With regard to osseous tissues, and irrespective of the carrier material used (osteoconductive or not), this matrix will provide the biomaterial-tissue hybrid with a unique combination of both osteoconductive and osteoinductive properties. In order to facilitate sufficient in vitro cell growth, matrix production and biomaterial-to-matrix bonding, the device should have bioactive or osteoconductive properties. The device according to this invention exhibits these properties, which makes it unique. Said bioactive or osteoconductive properties can be obtained via spontaneous calcification of the poly(oxyethylene)/poly(butylene terephtalate) (PEO/PBT) carrier material after immersion in culture medium or post-operatively, or it can be improved or enhanced via the use of a pre-operative calcification method utilising calcification solutions, or by the use of a bioactive filler material, such as needle-shaped carbonate-apatite or hydroxyapatite (i.e. calcium phosphates, bioactive glasses or glass ceramics).

The osteoinductive properties of the biomaterial-tissue hybrid is shown in the appending examples and is the result of either *in vitro* formed extracellular bone matrix with osteoinductive proteins, the presence of the osteogenic cells, or a combination of these two. In order to speed up proliferation, differentiation and extracellular matrix production by the cultured cells, the device could also be filled with osteoinductive factors, growth factors or other biologically active agents.

Besides osteoconductive properties, the device should have an open pore branching network, composed of a biocompatible and ideally biodegradable biomaterial, that is configured in an arrangement that provides for the diffusion of nutrients, oxygen and waste products. The device could be both biodegradable or non-biodegradable.

The structure of the material is a non-porous or partially or fully porous scaffold, three-dimensional matrix, or (elastic) film. Porosity can be obtained as a result of ordered fibres, fibre meshes (e.g. weaving) or open cell foams (e.g. as a result of salt addition), but is not limited to these processes.

The device according to the invention can be used for a variety of surgical treatments where osseous generation or regeneration is needed. These include all bone defects in orthopaedics, maxillofacial surgery, dentistry and any other disciplines where osseous (re)generation is required. The device can also be used for guided tissue regeneration membranes in e.g. dentistry.

The invention further pertains to the above described device, wherein said long-chain ester units constitute from 45 to 75% by weight of the copolyester.

The invention further pertains to the above described device, composed of a dense (non-porous) layer and one porous layer at one side or two porous layers at either side of said dense layer.

The invention further pertains to the above described device, having interconnected pores with a pore diameter of 50-800 µm, in particular 200-500 µm.

The invention further pertains to the above described device, having an increase in pore size from 10 to 800 µm from one side of the device to the other.

The invention further pertains to the above described device, in the form of a granulate or spheres with a size between 1 and 10, in particular between 2 and 5 mm.

The invention further pertains to the above described device, wherein said cells are selected from bone, bone marrow, cartilage, muscle tissue, fibrous tissue, skin, soft connective tissue or other connective tissues.

The invention further pertains to the above described device, wherein said cells have been induced to bone cells by differentiation from other cells.

The invention further pertains to the above described device, wherein said cells have formed a mineralised, partially mineralised or non-mineralised extracellular bone matrix.

The invention further pertains to the above described device, wherein a bone matrix has been formed by transformation of a cartilaginous matrix via endochondrial ossification by induction of said cultured cells or of a new population of cultured cells to form bone cells.

The invention further pertains to a method of producing a device for bone tissue engineering comprising the steps of :
(a) applying differentiated or undifferentiated bone-forming mammalian cells on a scaffold consisting of a segmented thermoplastic copolyester substrate;
(b) directly contacting said cells with a culture medium for a sufficient time to produce a mineralised or non-mineralised bone-like extracellular matrix;
(c) removing the substrate with the bone-like extracellular matrix from the culture medium.

The invention further pertains to the above described method for producing a device for bone tissue engineering, further comprising the step of immersing the substrate in a calcification solution before step (a) so as to improve the bioactivity, osteoconductivity or bone-bonding ability.

The invention further pertains to the above described method for producing a device for bone tissue engineering, further comprising the step of immersing the substrate in a solution of osteoinductive factors, growth hormones or other biologically active agents, before step (a) so as to improve the cellular proliferation, differentiation or extracellular matrix production by the cultured cells.

### EXAMPLE 1

### Rat bone marrow cell cultures on PEO/PBT copolymers

### Objective

The objective of this experiment was to examine the formation of a mineralized extracellular matrix on PEO/PBT copolymers.

### Materials

55/45 PEO/PBT; 2 mm thick dense discs
60/40 PEO/PBT copolymer; 0.8 mm thick dense discs
30/70 PEO/PBT copolymer; 0.8 mm thick dense discs

Prior to cell culture, all samples were rinsed in distilled water, dried at 37°C and sterilised by gamma irradiation.

### Method

### Cell culture

Bone marrow cells were isolated from the femora of young adult Wistar rats. In brief, the femora were washed 3 times in α-Minimum Essential Medium (α-MEM) containing ten times the normal concentration of antibiotics. The epiphyses were subsequently removed and each diaphysis was flushed out with 15 ml α-MEM containing 15% foetal bovine serum, antibiotics, 10 mM β-glycerophosphate, 50 µg/ml ascorbic acid and 10⁻⁸ M dexamethasone. The cell suspensions containing undifferentiated, differentiated and osteoprogenitor cells, were subsequently pooled and carefully resuspended by aspiration with a syringe and 21G needle. From this cell suspension, 200 µl droplets were inoculated onto the various materials, which were then incubated at 37°C/5% CO2 for several hours to allow cell attachment. Culture medium (as above) was subsequently added so that the samples were submerged; the culture plates were then replaced in the incubator. The cultures were refed three times weekly for 3-4 weeks.

Following the culture period, the samples were rinsed in phosphate buffered saline and fixed in 1.5% glutaraldehyde in 0.14 M cacodylate buffer for at least 24 hours. Following fixation, the samples were examined using light microscopy, scanning electron microscopy or transmission electron microscopy.

### Light microscopy (LM)

Following fixation, samples for LM were rinsed in 0.14 M cacodylate buffer, dehydrated through a graded ethanol series and embedded in glycol methacrylate resin. Following polymerisation, 2-3 µm sections were prepared, which were then stained with toluidine blue, Alizarin red or von Kossa.

### Scanning electron microscopy (SEM)

Following fixation, samples were rinsed in 0.14 M cacodylate buffer, dehydrated and critical point dried. Prior to either gold or carbon coating, the overlying cell multilayers were removed. The samples were subsequently examined using scanning electron microscopy, back scatter electron microscopy (BSEM) and X-ray microanalysis (XRMA).

### Transmission electron microscopy

Following fixation, samples were rinsed in 0.14 M cacodylate buffer, post-fixed in 1% osmium tetroxide/potassium ferrocyanate (1:1) in cacodylate buffer, dehydrated and embedded in Epon. Ultrathin sections were prepared and examined either unstained or after contrast staining with uranyl acetate and lead citrate.

### Backscatter electron microscopy (BSEM)

Following TEM sectioning, a selection of Epon tissue blocks were processed for BSEM. The blocks were polished with 4000 grit silicon carbide sandpaper, rinsed with 70% ethanol, dried and carbon-coated. The samples were examined using a scanning electron microscope in backscatter mode, at 20 kV.

### Results

Using light microscopy, it could be seen that the materials were biocompatible, in that no adverse tissue reactions were observed. Calcification, as a result of incubation in culture medium, was observed in the 55/45 and 60/40 materials although this phenomenon was not seen with the 30/70 copolymer. The presence of a mineralized extracellular matrix that had been produced by the cultured osteogenic cells, was demonstrated on the materials using Von Kossa and alizarin red staining techniques for phosphate and calcium respectively.

SEM revealed the deposition of globular, calcium and phosphate containing accretions on the surface of the materials. Mineralized collagen fibres were seen in close association with these globular strictures. In some areas, this mineralized extracellular matrix had fused to form a continuous layer.

TEM showed and backscatter electron microscopy revealed interfacial continuity between calcified structures in the 55/45 and 60/40 material surfaces and the in vitro formed mineralized extracellular matrix. With the 30/70 copolymer, mineralized extracellular matrix was seen in direct contact with the material surface although a continuity between the two was not observed.

### Conclusions

These results show that different compositions of PEO/PBT copolymers are biocompatible and can give rise to spontaneous calcification when immersed in tissue culture medium. Osteogenic cells cultured on the materials can form a bone-like mineralized extracellular matrix which forms a close continuity with the material in areas where it is calcified.

### EXAMPLE 2

### Rat bone marrow cell cultures on precalcified PEO/PBT copolymers and PEO/PBT copolymer composites with AW glass ceramic and hydroxyapatite

### Objective

The objective of this experiment was to examine the formation of a bone-like mineralized extracellular matrix on precalcified PEO/PBT copolymers and PEO/PBT composites.

### Materials

55/45 PEO/PBT precalcified in calcification solution
55/45 PEO/PBT / AW glass ceramic composite; 5, 9, 20 and 50% AW glass
55/45 PEO/PBT / amorphous HA composite; 10 and 20% aHA

Prior to cell culture, all samples were rinsed in distilled water, dried at 37°C and sterilised by gamma irradiation.

### Method

### Precalcification of PEO/PBT copolymers in calcium chloride/Na₂HPO₄ solution

Materials for precalcification were incubated in 1M calcium chloride for 3 days at room temperature, rinsed briefly in distilled water and dried at 37°C. They were subsequently incubated in 1M Na₂HPO₄ for 3 days at room temperature, rinsed briefly in distilled water, dried at 37°C and gamma irradiated prior to cell culture.

### Scanning electron microscopy (SEM) of the starting materials

Cross-sections of the composite plates were sputter-coated with carbon and were examined using a scanning electron microscope at an accelerating voltage of 15 kV.

### Cell culture

Bone marrow cells were isolated from the femora of young adult Wistar rats. In brief, the femora were washed 3 times in α-Minimum Essential Medium (α-MEM) containing ten times the normal concentration of antibiotics. The epiphyses were subsequently removed and each diaphysis was flushed out with 15ml α-MEM containing 15% foetal bovine serum, antibiotics, 10mM β-glycerophosphate, 50µg/ml ascorbic acid and 10⁻⁸M dexamethasone. The suspensions, containing undifferentiated, differentiated and osteoprogenitor cells, were subsequently pooled and carefully resuspended by aspiration with a syringe and 21G needle. From this cell suspension, 200µl droplets were inoculated onto the various materials, which were then incubated at 37°C/5% CO2 for several hours to allow cell attachment. Culture medium (as above) was subsequently added so that the samples were submerged. The cultures were refed three times weekly and were maintained for 2 and 3 weeks. Following the culture period, the samples were rinsed in phosphate buffered saline and fixed in 1.5% glutaraldehyde in 0.14M cacodylate buffer for at least 24 hours. Following fixation, the samples were examined using light microscopy and transmission electron microscopy.

### Light microscopy (LM) and transmission electron microscopy (TEM)

Following fixation, samples were rinsed in 0.14M cacodylate buffer, post-fixed in 1% osmium tetroxide/potassium ferrocyanate (1:1) in cacodylate buffer, dehydrated and embedded in Epon. For light microscopy, 2-3 µm sections were prepared, which were then stained with toluidine blue or the calcium specific stain Alizarin red. For TEM, ultrathin sections were prepared, contrast stained with uranyl acetate and lead citrate and examined in a transmission electron microscope at an accelerating voltage of 80 kV.

### Backscatter electron microscopy (BSEM) and X-ray microanalysis (XRMA)

Following TEM sectioning, the Epon tissue blocks were processed for BSEM and XRMA. The blocks were polished with 4000 grit silicon carbide sandpaper, rinsed with 70% ethanol, dried and carbon-coated. The samples were examined using a scanning electron microscope in backscatter mode, with an X-ray microanalysis unit.

### Results

Scanning electron microscopy of the composite starting materials showed a random distribution of both the AW glass ceramic and the aHA particles throughout the copolymer matrix. Light microscopy revealed that the materials were biocompatible, in that no adverse tissue reactions were observed. Cellular nodules were seen on the materials and alizarin red staining revealed the presence of a mineralized extracellular matrix on the PEO/PBT / AW glass composites, with the exception of the 2 week culture on the PEO/PBT / 5% AW glass composite. In case of the precalcified PEO/PBT copolymer, a continuous layer of mineralized extracellular matrix was observed on the surface of the material. TEM showed a close contact between the mineralized extracellular matrix and the material surfaces. XRMA demonstrated the presence of calcification in all materials, except the 5% AW glass composite after 2 weeks in culture.

### Conclusions

These results show that biocompatible, precalcified copolymers, or copolymer composites can be produced, on which bone marrow cells can be grown that have the capacity to form a bone-like mineralized extracellular matrix. The close relation between calcified areas in the precalcified copolymer or copolymer composites and the bone-like mineralized extracellular matrix, indicates the osteoinductive character of these materials.

### EXAMPLE 3

### Cell culture system for in vivo bone tissue engineering in (porous) ceramics and polymeric biomaterials

### Objective

To examine whether in vitro formed mineralized bone-like tissue, when formed in (porous) ceramic and polymeric templates, exhibits osteoconductive and osteoinductive properties when implanted in non-osseous sites, in vivo.

### Materials

- Porous hydroxyapatite; 400µm pore diameter, 30% porosity; sintered at 1300°C for 96 hours
- Porous 70/30 PEO/PBT copolymer sheet (PEO = 1000 Dalton molecular weight), 2mm thick dense layer and a porous layer, 300 - 600µm pore diameter, (i) non-precalcified and (ii) precalcified with Simulated Body Fluid which mimics the inorganic composition of tissue fluids;

### Method

### Cell culture

Bone marrow cells were isolated from the femora of young adult Fischer rats. The femora were washed 3 times in α-Minimum Essential Medium (α-MEM) containing ten times the normal concentration of antibiotics. The epiphyses were subsequently removed and each diaphysis was flushed out with 6 ml α-MEM containing 15% foetal bovine serum, antibiotics, 10 mM β-glycerophosphate, 50 µg/ml ascorbic acid and 10⁻⁸ M dexamethasone. The cell suspensions were subsequently pooled and carefully resuspended by aspiration with a syringe and 21G needle. These primary cells were either directly seeded onto the various samples (600 µl/sample) or were cultured in tissue culture flasks for 5-7 days until near confluency was reached. For the latter, the cells were rinsed in phosphate buffered saline on reaching confluency, followed by trypsinisation to detach the cells from the culture surface. These sub-cultured cells were then counted and seeded onto the materials at a concentration of 1-2 x 10⁵ cells per sample. All cultures were maintained in α-MEM medium as described above, in a humidified atmosphere of 95% air/5% CO₂ at 37°C. The medium was changed after the first 24 hours to remove non-adherent cells and was further refreshed three times weekly. After 4 weeks in vitro, a selection of samples were implanted subcutaneously in rats (see below); the remaining samples were cultured for a further one to four weeks. Control materials were placed in cell free culture medium in order to examine possible medium mediated alterations of the samples.

### Implantation procedure

The selected samples were implanted in the backs of 250-300 gram male albino Fischer rats. The rats were anaesthetised, shaved and cleaned with 10% ethanol/iodine. Two subcutaneous pockets were created on each side of the spine and one implant was inserted in each pocket. Survival periods included 1 and 4 weeks, after which time the samples were retrieved and evaluated using light microscopy and scanning electron microscopy.

### Fixation

Following the implantation periods, the rats were euthanised, and the samples were retrieved and fixed in 1.5% glutaraldehyde in 0.14M cacodylate buffer, pH 7.4, for at least 24 hours. In vitro samples were fixed in similar fixative. Following fixation, the samples were processed for the various evaluation techniques.

### Light microscopy (LM)

Following fixation, samples for LM were rinsed in 0.14 M cacodylate buffer, dehydrated through a graded ethanol series and embedded in methyl methacrylate resin. Following polymerisation, undecalcified 10 µm thick sections were prepared using a modified inner-lock diamond saw; the section were stained with methylene blue and basic fuchsin.

The remainder of the LM tissue blocks were subsequently processed for backscatter electron microscopy.

### Backscatter electron microscopy (BSEM)

Following sectioning for LM, the tissue blocks were polished with 4000 grit silicon carbide sandpaper, rinsed with 70% ethanol, dried and carbon-coated. The samples were examined using a scanning electron microscope in backscatter mode, at 20 kV.

### Scanning electron microscopy (SEM)

Following fixation, samples were rinsed in 0.14 M cacodylate buffer, dehydrated and critical point dried. The samples were subsequently examined using a scanning electron microscopy at 15 kV.

### Results

### Hydroxyapatite

After 4 weeks in vitro, a mineralized extracellular matrix had formed along most of the outer surface of the hydroxyapatite and after 8 weeks, the thickness of this layer had increased. After 4 weeks in vitro, followed by 4 weeks in vivo, bone formation was observed, both on the outer surface and in the pores of the material. This bone tissue was clearly distinguishable from the in vitro formed mineralized matrix, in that it had an organised structure with, for example, osteoblast seams and osteocytes in lacunae. In the control samples, without cultured cells, bone formation was not observed.

### Non-precalcified and precalcified PEO/PBT copolymers

After 4 weeks in vitro, the formation of a mineralized extracellular matrix was observed in both materials. In the control samples, without cells, abundant calcification of the materials was observed at all evaluation times.

After 4 weeks in vitro, followed by 4 weeks in vivo, bone formation was observed in both the non-precalcified and precalcified materials. As with the HA, this bone tissue was morphologically more mature than the in vitro formed mineralized matrix. Mineralized extracellular matrix was not observed in any of the control cultures (without cells), which confirms the non-osteoinductive character of the material itself, while the material-cultured bone-like tissue hybrid exhibits osteoinductive properties. Degradation of the porous polymer was seen, in that small fragments were visible. This degradation did not give rise to an inflammatory response or any adverse tissue reactions.

### Conclusions

The results show that rat bone marrow cells can be cultured in porous degradable biomaterials to produce an osteoinductive, osteoconductive hybrid material. Although the materials themselves do not give rise to osteoinduction, the presence of an in vitro formed bone-like extracellular matrix results in osteoinduction and de novo bone formation. This suggests that these materials can be used as carriers for bone tissue engineering. With the copolymer, a flexible autogenous cultured bone graft can be produced in vitro for implantation purposes.

## Claims

1. A device for bone tissue engineering comprising a scaffold material consisting of a bioactive, osteoconductive and bone-bonding segmented thermoplastic copolyesterether and in vitro cultured osteogenic or osteoprogenitor cells.

2. The device of claim 1, wherein said copolyesterether consists essentially of a multiplicity of recurring long-chain ester units and short-chain ester units, the long-chain ester units comprising from 35 to 80% by weight of the copolyesterether and being represented by the formula
-OLO-CO-R-CO- or -OLO-CO-
and the short-chain ester units being represented by the formula
-OEO-CO-R-CO- and/or -O-Q-CO-
wherein
L is a divalent group remaining after removal of terminal hydroxyl groups from a poly(oxyalkylene) glycol with an average molecular weight of between 300 and 3000;
R is a divalent group remaining after removal of carboxyl groups from a dicarboxylic acid having a molecular weight of less than 300;
Q is an alkylene group having 1-6 carbon atoms and/or a cyclohexylene of phenylene group; and
E is an alkylene group having 2-6 carbon atoms.

3. The device according to claim 2, wherein said long-chain ester units constitute from 45 to 75% by weight of the copolyester.

4. The device according to claim 3, wherein said long-chain ester units are poly(oxyethylene) units and said short-chain ester units are poly(butylene terephthalate) units.

5. The device of claim 1, composed of a dense (non-porous) layer and one porous layer at one side or two porous layers at either side of said dense layer.

6. The device of claim 1, having interconnecting pores with a pore diameter of 50-800 µm, in particular 200-500 µm.

7. The device of claim 1, having an increase in pore size from 10 to 800 µm from one side of the device to the other.

8. The device of claim 1, wherein said copolyester has been precalcified by immersion in a calcification fluid.

9. The device of claim 1, in the form of a granulate or spheres with a size between 1 and 10, in particular between 2 and 5 mm.

10. The device of claim 1, further comprising osteoinductive factors, growth hormones, or other biologically active agents.

11. The device of claim 1, wherein said cells are selected from bone, bone marrow, cartilage, muscle tissue, fibrous tissue, skin, soft connective tissue or other connective tissues.

12. The device of claim 11, wherein said cells have been induced to bone cells by differentiation from other cells.

13. The device of claim 1, wherein said cells have formed a mineralised, partially mineralised or non-mineralised extracellular bone matrix.

14. The device of claim 1, wherein a bone matrix has been formed by transformation of a cartilaginous matrix via endochondrial ossification by induction of said cultured cells or of a new population of cultured cells to form bone cells.

15. A method of producing a device for bone tissue engineering comprising the steps of:
(a) applying differentiated or undifferentiated bone-forming mammalian cells on a scaffold consisting of a segmented thermoplastic copolyester substrate;
(b) directly contacting said cells with a culture medium for a sufficient time to produce a mineralised or non-mineralised bone-like extracellular matrix;
(c) removing the substrate with the bone-like extracellular matrix from the culture medium.

16. The method of claim 15, further comprising the step of immersing the substrate in a calcification solution before step (a) so as to improve the bioactivity, osteoconductivity or bone-bonding ability.

17. The method of claim 15, further comprising the step of immersing the substrate in a solution of osteoinductive factors, growth hormones or other biologically active agents, before step (a) so as to improve the cellular proliferation, differentiation or extracellular matrix production by the cultured cells.

## Patentansprüche

1. Vorrichtung zur künstlichen Herstellung von Knochengewebe, die ein Gerüstmaterial umfasst, das aus einem bioaktiven, osteokonduktiven und knochenbindenden segmentierten thermoplastischen Copolyesterether und in vitro kultivierten knochenbildenden Zellen oder Knochenvorläuferzellen besteht.

2. Vorrichtung gemäß Anspruch 1, wobei der Copolyesterether im wesentlichen aus einer Vielzahl von langkettigen Ester-Repetiereinheiten und kurzkettigen Ester-Repetiereinheiten besteht, wobei die langkettigen Estereinheiten 35 bis 80 Gew.-% des Copolyesterethers umfassen und durch die Formel
-OLO-CO-R-CO- oder -OLO-CO-
dargestellt werden und die kurzkettigen Estereinheiten durch die Formel
-OEO-CO-R-CO- und/oder -O-Q-CO-
dargestellt werden, wobei
L eine zweiwertige Gruppe ist, die nach der Entfernung terminaler Hydroxygruppen von einem Polyoxyalkylenglycol mit einem mittleren Molekulargewicht zwischen 300 und 3000 zurückbleibt;
R eine zweiwertige Gruppe ist, die nach der Entfernung der Carboxygruppen von einer Dicarbonsäure mit einem Molekulargewicht von weniger als 300 zurückbleibt;
Q eine Alkylengruppe mit 1 bis 6 Kohlenstoffatomen und/oder eine Cyclohexylen- oder Phenylengruppe ist; und
E eine Alkylengruppe mit 2 bis 6 Kohlenstoffatomen ist.

3. Vorrichtung gemäß Anspruch 2, wobei die langkettigen Estereinheiten 45 bis 75 Gew.-% des Copolyesters ausmachen.

4. , Vorrichtung gemäß Anspruch 3, wobei es sich bei den langkettigen Estereinheiten um Polyoxyethyleneinheiten und bei den kurzkettigen Estereinheiten um Polybutylenterephthalateinheiten handelt.

5. Vorrichtung gemäß Anspruch 1, die aus einer dichten (nichtporösen) Schicht und einer porösen Schicht auf einer Seite oder zwei porösen Schichten auf beiden Seiten der dichten Schicht besteht.

6. Vorrichtung gemäß Anspruch 1, die zusammenhängende Poren mit einem Porendurchmesser von 50 bis 800 µm, insbesondere 200 bis 500 µm, aufweist.

7. Vorrichtung gemäß Anspruch 1, die eine Zunahme der Porengröße von 10 bis 800 µm von einer Seite der Vorrichtung bis zur anderen aufweist.

8. Vorrichtung gemäß Anspruch 1, wobei der Copolyester durch Eintauchen in eine Calzifizierungsflüssigkeit vorcalcifiziert wurde.

9. Vorrichtung gemäß Anspruch 1 in Form eines Granulats oder von Kugeln mit einer Größe zwischen 1 und 10, insbesondere zwischen 2 und 5 mm.

10. Vorrichtung gemäß Anspruch 1, die weiterhin osteoinduktive Faktoren, Wachstumshormone oder andere biologisch aktive Agentien umfasst.

11. Vorrichtung gemäß Anspruch 1, wobei die Zellen aus Knochen-, Knochenmark-, Knorpel-, Muskelgewebe-, Fasergewebe-, Haut-, weichen Bindegewebs- oder anderen Bindegewebszellen ausgewählt sind,

12. Vorrichtung gemäß Anspruch 11, wobei die Zellen durch Differenzierung aus anderen Zellen zu Knochenzellen induziert wurden,

13. Vorrichtung gemäß Anspruch 1, wobei die Zellen eine mineralisierte, partiell mineralisierte oder nichtmineralisierte extrazelluläre Knochenmatrix gebildet haben.

14. Vorrichtung gemäß Anspruch 1, wobei eine Knochenmatrix durch Transformation einer Knorpelmatrix über endochondriale Ossifizierung durch Induktion der kultivierten Zellen oder einer neuen Population kultivierter Zellen unter Bildung von Knochenzellen gebildet wurde.

15. Verfahren zur Herstellung einer Vorrichtung zur künstlichen Herstellung von Knochengewebe, das die folgenden Schritte umfasst;
(a) Aufbringen differenzierter oder undifferenzierter knochenbildender Säugerzellen auf ein Gerüst, das aus einem segmentierten thermoplastischen Copolyester-Substrat besteht;
(b) direktes In-Kontakt-Bringen der Zellen mit einem Kulturmedium während einer ausreichenden Zeit, so dass eine mineralisierte oder nichtmineralisierte knochenartige extrazelluläre Matrix entsteht;
(c) Entfernen des Substrats mit der knochenartigen extrazellulären Matrix aus dem Kulturmedium.

16. Verfahren gemäß Anspruch 15, das weiterhin den Schritt des Eintauchens des Substrats in eine Calzifizierungslösung vor Schritt (a) umfasst, um die Bioaktivität, Osteokonduktivität oder Knochenbildungsfähigkeit zu verbessern.

17. Verfahren gemäß Anspruch 15, das weiterhin den Schritt des Eintauchens des Substrats in eine Lösung von osteoinduktiven Faktoren, Wachstumshormonen oder anderen biologisch aktiven Agentien vor Schritt (a) umfasst, so dass die Zellproliferation, Differenzierung oder extrazelluläre Matrixproduktion durch die kultivierten Zellen verbessert wird.

## Revendications

1. Dispositif pour l'ingénierie du tissu osseux comprenant un matériau de support consistant en un copolyesteréther thermoplastique segmenté bioactif, ostéoconducteur et se liant aux os et des cellules ostéogènes ou ostéoprogénitrices cultivées in vitro.

2. Dispositif selon la revendication 1, où ledit copolyesteréther consiste essentiellement en une multiplicité d'unités esters à longue chaîne et d'unités esters à courte chaîne répétées, les unités esters à longue chaîne constituant de 35 à 80 % en masse du copolyesteréther et étant représentées par la formule
-OLO-CO-R-CO- ou -OLO-CO-
et les unités esters à courte chaîne étant représentées par la formule
-OEO-CO-R-CO- et/ou -O-Q-CO-
où
L est un groupe divalent restant après le retrait des groupes hydroxyle terminaux d'un poly(oxyalkylène)glycol ayant une masse moléculaire moyenne située entre 300 et 3 000 ;
R est un groupe divalent restant après le retrait des groupes carboxyle d'un acide dicarboxylique ayant une masse moléculaire inférieure à 300 ;
Q est un groupe alkylène ayant 1 à 6 atomes de carbone et/ou un groupe cyclohexylène ou phénylène ; et
E est un groupe alkylène ayant 2-6 atomes de carbone.

3. Dispositif selon la revendication 2, où lesdites unités esters à longue chaîne constituent de 45 à 75 % en masse du copolyester.

4. Dispositif selon la revendication 3, où lesdites unités esters à longue chaîne sont des unités de poly(oxyéthylène) et lesdites unités esters à courte chaîne sont des unités de poly(butylènetéréphtalate).

5. Dispositif selon la revendication 1, composé d'une couche dense (non poreuse) et d'une couche poreuse d'un côté ou de deux couches poreuses de chaque côté de ladite couche dense.

6. Dispositif selon la revendication 1, ayant des pores reliés entre eux ayant un diamètre de pores de 50-800 µm, en particulier de 200-500 µm.

7. Dispositif selon la revendication 1, ayant une augmentation de la taille des pores de 10 à 800 µm d'un côté du dispositif à l'autre.

8. Dispositif selon la revendication 1, où ledit copolyester a été précalcifié par immersion dans un fluide de calcification.

9. Dispositif selon la revendication 1 sous forme de granulés ou de sphères ayant une taille située entre 1 et 10, en particulier entre 2 et 5 mm.

10. Dispositif selon la revendication 1 comprenant en outre des facteurs ostéoinducteurs, des hormones de croissance ou d'autres agents biologiquement actifs.

11. Dispositif selon la revendication 1 où lesdites cellules sont choisies parmi les os, la moelle osseuse, le cartilage, le tissu musculaire, le tissu fibreux, la peau, le tissu conjonctif mou ou d'autres tissus conjonctifs.

12. Dispositif selon la revendication 11 où lesdites cellules ont été amenées à former des cellules osseuses par différenciation à partir d'autres cellules.

13. Dispositif selon la revendication 1 où lesdites cellules ont formé une matrice osseuse extracellulaire minéralisée, partiellement minéralisée ou non minéralisée.

14. Dispositif selon la revendication 1 où une matrice osseuse a été formée par transformation d'une matrice cartilagineuse par ossification endochondriale par le fait que lesdites cellules cultivées ou une nouvelle population de cellules cultivées ont été amenées à former des cellules osseuses.

15. Procédé de production d'un dispositif pour l'ingénierie du tissu osseux comprenant les étapes de :
(a) application de cellules de mammifère formant des os différenciées ou non différenciées sur un support consistant en un substrat de copolyester thermoplastique segmenté ;
(b) mise en contact directe desdites cellules avec un milieu de culture pendant une durée suffisante pour produire une matrice extracellulaire analogue aux os minéralisée ou non minéralisée ;
(c) retrait du substrat avec la matrice extracellulaire analogue aux os du milieu de culture.

16. Procédé selon la revendication 15 comprenant en outre l'étape d'immersion du substrat dans une solution de calcification avant l'étape (a) de manière à améliorer la bioactivité, l'ostéoconductivité ou l'aptitude de liaison aux os.

17. Procédé selon la revendication 15 comprenant en outre l'étape d'immersion du substrat dans une solution de facteurs ostéoinducteurs, d'hormones de croissance ou d'autres agents biologiquement actifs avant l'étape (a) de manière à améliorer la prolifération cellulaire, la différenciation cellulaire ou la production d'une matrice extracellulaire par les cellules cultivées.
